# EUROPEAN PATENT APPLICATION

(11) **EP 4 851 605 A1**
(43) Date of publication of application: **09.12.2026**
(21) Application number: 25749225.6
(22) Date of filing: 24.01.2025
(51) Int. Cl.: G01N 21/03, G01N 21/25, G01N 33/72, G01N 21/27, G01N 21/31

(54) **APPARATUS FOR MEASURING HEMOGLOBIN AND METHOD FOR MEASURING HEMOGLOBIN CONCENTRATION BY USING SAME**

(30) Priority: 31.01.2024 KR 20240015230; 31.01.2024 KR 20240000206 U
(71) Applicant: Genbody Inc., Cheonan-si, Chungcheongnam-do 31077 (KR)
(72) Inventor: CHONG, Chomkyu, Cheonan-si Chungcheongnam-do 31161 (KR); MYEONG, Hyeonkoon, Daejeon 34140 (KR); PARK, Jongpil, Sejong 30100 (KR); LEE, Hyuntaek, Cheonan-si Chungcheongnam-do 31079 (KR); YOO, Junwon, Gwangmyeong-si Gyeonggi-do 14345 (KR)
(74) Representative: Cabinet Becker et Associés
(86) International application number: PCT/KR2025/099143
(87) International publication number: WO 2025/165186

(57) **Abstract**

The present disclosure relates to a hemoglobin measuring device and a method of measuring hemoglobin concentration using the same.

## Description

### [Technical Field]

The present disclosure relates to a hemoglobin measuring device and a method of measuring hemoglobin concentration using the same, and more particularly, to a method of measuring the concentration of hemoglobin in the blood collected by a cuvette.

### [Background Art]

The contents described in this section merely provide background information for the present disclosure and do not constitute conventional technologies.

Hemoglobin is a protein found in blood, and plays an important role in oxygen transport. Thus, a lack of hemoglobin may cause health problems such as anemia. Therefore, measuring the concentration of hemoglobin in the blood is a very important task for some patients. There are many simple hemoglobin measuring devices on the market for easy hemoglobin measurement.

Such simple hemoglobin measuring devices employ a method where a user makes an incision in a part of their body using a tool such as a lancet to collect blood, drops the collected blood into a tray of the hemoglobin measuring device, and uses this blood to measure the concentration of hemoglobin. In this case, the user may experience inconvenience as excessive amounts of their blood are collected.

Accordingly, a method is often used in which the user collects its own blood using a tool called a cuvette and inserts it into a hemoglobin measuring device.

Conventional cuvettes are usually made of a hydrophilic material such as glass. Glass cuvettes have a complex manufacturing process, are expensive to manufacture, and can cause injury to the user if they are dropped and broken. In addition, conventional cuvettes have problems that air bubbles are captured together when collecting blood, which decreases the accuracy of blood analysis and increases a high defect rate during manufacturing.

Furthermore, conventional hemoglobin measuring devices use optical elements, resulting in a large size because a minimum light path must be secured. Thus, there are limitations in portability and movement.

In addition, in the case of these commercially available simple hemoglobin measuring devices, light emitting elements in the measuring devices may deteriorate due to numerous repeated uses. Deterioration of the light emitting elements reduces the output of emitted light, causing the inaccurate measurement of hemoglobin concentration. Specifically, as the intensity of light output from the light emitting elements decreases, the hemoglobin concentration may be measured higher than actual. In the case of anemic patients, there is a problem that hemoglobin concentrations higher than their own actual hemoglobin concentrations may be measured, thereby leading to missed appropriate measures and fatal consequences for the patients.

In addition, commercially available simple hemoglobin measuring devices use optical elements, so there is a problem of large size because a minimum light path must be secured. Thus, there are restrictions in portability and movement.

### [Disclosure]

### [Technical Problem]

Accordingly, the present disclosure aims to provide a cuvette that is low in manufacturing cost and has high productivity.

In addition, the present disclosure aims to provide a cuvette capable of being safely used by users.

In addition, the present disclosure aims to provide a cuvette having high analysis accuracy.

In addition, the present disclosure aims to provide a cuvette having excellent sample collection ability while being inexpensive to manufacture.

In addition, the present disclosure aims to provide a cuvette having a low defect rate during manufacturing.

In addition, the present disclosure aims to provide a user-friendly cuvette.

In addition, the present disclosure aims to provide a hemoglobin measuring device having good portability and mobility.

In addition, the present disclosure aims to provide a method of measuring the concentration of hemoglobin with high accuracy even when deterioration occurs in a light emitting element.

In addition, the present disclosure aims to provide a hemoglobin measuring device that can be space-efficiently designed, thus making it easy to carry and move.

The problems to be solved by the present invention are not limited to the problems mentioned above, and other problems not mentioned will be clearly understood by those skilled in the art from the description below.

### [Technical Solution]

According to a first embodiment of the present disclosure, there is provided a cuvette including: a handle 110 configured to grasp; a suction unit 120 formed integrally with the handle 110, having an open upper surface and configured to suck a sample through an opening 121 formed at one end; a hydrophilic film 140 attached to the suction unit 120 to form an accommodation space together with the suction unit 120; and an adhesive layer 130 placed between the hydrophilic film 140 and an attachment surface 125 of the suction unit 120 to attach both of them.

Preferably, the suction unit 120 according to the first embodiment of the present disclosure is made of one or more materials selected from the group consisting of polystyrene (PS), polyester (PES), polyethylene terephthalate (PET), and polymethyl methacrylate (PMMA).

Further, preferably, the suction unit 120 according to the first embodiment of the present disclosure includes: an inclined surface 122 formed to extend from the opening 121 toward the inside of the cuvette 100; an inner bottom surface 123 formed to extend from an endpoint of the inclined surface 122 toward the inside of the cuvette 100; a circular protrusion 124 formed to protrude upward from the inner bottom surface 123; and an air discharge portion 126 formed to penetrate the suction unit 120.

Further, preferably, the height of the opening 121 according to the first embodiment of the present disclosure, that is, the distance (d1) between the hydrophilic film 140 and a starting point of the inclined surface 122 ranges from 0.3 mm to 1.0 mm, the distance (d2) between the hydrophilic film 140 and the endpoint of the inclined surface 122 ranges from 0.2 mm to 0.5 mm, and the distance (d3) between the protrusion 124 and the hydrophilic film 140 ranges from 0.05 mm to 0.2 mm.

According to a second or third embodiment of the present disclosure, there is provided a cuvette 200 or 300 configured to capture a sample, the cuvette 200 or 300 including: a handle 210 or 310 configured to grasp; and a suction unit 220 or 320 formed integrally with the handle 210 or 310 and configured to suck a sample through an opening 221 or 321 formed at one end, wherein the suction unit 220 or 320 has a shape in which the upper surface, lower surface, and both side surfaces are closed, and at least a part of the suction unit 220 or 320 is made of a hydrophilic bio-fiber material.

Preferably, the suction unit 220 according to the second embodiment of the present disclosure includes: a first inclined surface 222a formed at one end of an inner bottom surface 223 of the suction unit 220 and extending from the opening 221 toward the inside of the cuvette 200; a second inclined surface 222b formed at one end of an inner upper surface 225 of the suction unit 220 and extending from the opening 221 toward the inside of the cuvette 200; a circular protrusion 224 formed to protrude upward from the inner bottom surface 223; and an air discharge portion 226 formed to penetrate the upper surface of the suction unit 220.

Further, preferably, the suction unit 320 according to the third embodiment of the present disclosure includes: a first inclined surface 322a formed at one end of an inner bottom surface 323 of the suction unit 320 and extending from the opening 321 toward the inside of the cuvette 300; a second inclined surface 322b formed at one end of an inner upper surface 325 of the suction unit 320 and extending from the opening 321 toward the inside of the cuvette 300; a first circular protrusion 324a formed to protrude upward from the inner bottom surface 323; a second circular protrusion 324b formed to protrude downward from the inner upper surface 325; and an air discharge portion 326 formed to penetrate the upper surface of the suction unit 320.

According to another embodiment of the present disclosure, there is provided a hemoglobin measuring device using the cuvette 100, 200 or 300, the hemoglobin measuring device including: a first light emitting unit 12 configured to emit light downward; a second light emitting unit 14 configured to emit light rightwards; a first light receiving unit 32 arranged parallel to the second light emitting unit 14 at the right side of the second light emitting unit 14; a second light receiving unit 34 arranged parallel to the first light emitting unit 12 at the lower side of the first light emitting unit 12; and a beam splitter 20 placed at the point where a path from the first light emitting unit 12 to the second light receiving unit 34 intersects with the path from the second light emitting unit 14 to the first light receiving unit 32, wherein the cuvette 100, 200, or 300 is placed between the beam splitter 20 and the second light receiving unit 34.

Preferably, the wavelength of light emitted from the first light emitting unit 12 ranges from 525 nm to 530 nm, and the wavelength of light emitted from the second light emitting unit 14 is 840 nm.

According to another embodiment of the present disclosure, there is provided a method of measuring hemoglobin concentration using a hemoglobin measuring device 1 comprising a first light emitting unit 12, a second light emitting unit 14, a first light receiving unit 32 receiving a part of light emitted from the first light emitting unit 12 and a part of light emitted from the second light emitting unit 14, and a second light receiving unit 34 receiving another part of light emitted from the first light emitting unit 12 and a part of light emitted from the second light emitting unit 14, the method including: (A) a step of calculating a first correction criterion and a second correction criterion using the hemoglobin measuring device 1 used a number of times less than a preset number of times; and (B) a step of measuring the concentration of hemoglobin in blood using the hemoglobin measuring device 1 used more than a preset number of times.

Preferably, the step (A) according to another embodiment of the present disclosure includes: (a1) a step of allowing the first light receiving unit 32 to measure the absorbance (A1_REF) of a wavelength (first wavelength) of light emitted from the first light emitting unit 12 and the absorbance (A2_REF) of a wavelength (second wavelength) of light emitted from the second light emitting unit 14; (a21) a step of repeating the step (a1) several times; and (a22) a step of allowing a control unit 40 to calculate a first correction criterion which is an average of the absorbance (A1_REF) of the first wavelength and a second correction criterion which is an average of the absorbance (A2_REF) of the second wavelength.

Further, preferably, the step (B) according to another embodiment of the present disclosure includes: (b1) a step of allowing each of the first light emitting unit 12 and the second light emitting unit 14 to emit light; (b2) a step of allowing the first light receiving unit 32 to measure the absorbance (A1_REF) of the first wavelength and the absorbance (A2_REF) of the second wavelength and allowing the second light receiving unit 34 to measure the absorbance (A1) of the first wavelength and the absorbance (A2) of the second wavelength; (b3) a step of allowing the control unit 40 to calculate corrected absorbances (A1_corrected and A2_corrected) of the second light receiving unit 34 using the first correction criterion calculated in the step (a22) and the absorbances (A1_REF and A2_REF) of the first light receiving unit 32 measured in the step (b2); and (b4) a step of calculating the concentration of hemoglobin of a sample in the cuvette 100 using the corrected absorbances (A1_corrected and A2_corrected) of the second light receiving unit 34.

Further, preferably, in the step (b3) according to another embodiment of the present disclosure, the corrected absorbances (A1_corrected and A2_corrected) of the second light receiving unit 34 are calculated using Equation 1 and Equation 3.

Further, preferably, in the step (b4) according to another embodiment of the present disclosure, the concentration of hemoglobin of the sample in the cuvette 100 is calculated using the corrected absorbances (A1_corrected and A2_corrected) of the second light receiving unit 34 and Equation 3.

Further, preferably, according to another embodiment of the present disclosure, the first wavelength ranges from 525 nm to 530 nm, and the second wavelength is 840 nm.

### [Advantageous Effects]

As described above, according to the first embodiment of the present disclosure, the cuvette has an effect of being capable of mass production and having a low production cost, thereby achieving excellent productivity.

In addition, the cuvettes according to all embodiments of the present disclosure has an effect of allowing a user to use them safely without fear of breakage.

In addition, the cuvette according to all embodiments of the present disclosure has an effect of being able to discharge air bubbles mixed during sample suction or blood collection to the outside of the cuvette, thereby improving analysis accuracy.

In addition, since the cuvette according to all embodiments of the present disclosure is configured to capture only a small amount of blood, there is no need for the user to collect an excessive amount of their own blood, resulting in a user-friendly effect.

In addition, the cuvette according to the first embodiment of the present disclosure has a portion formed by plastic injection molding and the other portion formed by a hydrophilic material, thereby reducing manufacturing costs while achieving excellent capturing ability.

In addition, the cuvettes according to the second and third embodiments of the present disclosure are injection-molded using a plastic including a hydrophilic biomaterial, and thus have excellent collection ability while being inexpensive to manufacture.

In addition, the hemoglobin measuring device according to another embodiment of the present disclosure is designed to be space-intensive because each light-emitting unit is arranged at 90 degrees and each light-receiving unit is also arranged at 90 degrees, thereby enhancing portability and mobility.

Further, as described above, the method of measuring hemoglobin concentration according to another embodiment of the present disclosure has an effect of measuring the concentration of hemoglobin with high accuracy by taking into account a decrease in output due to deterioration of a light-emitting element.

In addition, the measuring device used in the method of measuring hemoglobin concentration according to another embodiment of the present disclosure has an effect of being designed in a space-intensive manner because each light-emitting part is arranged at 90 degrees and each light-receiving part is also arranged at 90 degrees.

### [Brief Description of the Drawing]

FIG. 1 is a block diagram of a hemoglobin measuring device according to an embodiment of the present disclosure.
FIG. 2 illustrates the interior of a hemoglobin measuring device according to an embodiment of the present disclosure.
FIG. 3 illustrates the configuration of a hemoglobin measuring device according to the first embodiment of the present disclosure.
FIG. 4A is a front perspective view of a cuvette used for hemoglobin measurement according to the first embodiment of the present disclosure.
FIG. 4B is a bottom perspective view of a cuvette used for hemoglobin measurement according to the first embodiment of the present disclosure.
FIG. 5 is an exploded perspective view of a cuvette according to the first embodiment of the present disclosure viewed from the front view.
FIG. 6 is an enlarged cross-sectional view of a suction unit taken along the line I-I' in FIG. 4A of the present disclosure.
FIG. 7 is a usage status view of a cuvette according to the first embodiment of the present disclosure.
FIG. 8A is a front perspective view of a cuvette according to the second embodiment of the present disclosure.
FIG. 8B is a bottom perspective view of a cuvette according to the second embodiment of the present disclosure.
FIG. 9 is a left side view of a cuvette according to the second embodiment of the present disclosure.
FIG. 10A is a front perspective view of a cuvette according to the third embodiment of the present disclosure.
FIG. 10B is a bottom perspective view of a cuvette according to the third embodiment of the present disclosure.
FIG. 11 is a left side view of a cuvette according to the third embodiment of the present disclosure.
FIG. 12 is a flow chart of a method of measuring hemoglobin using a hemoglobin measuring device according to an embodiment of the present disclosure.

### [Mode for Carrying out the Invention]

Hereinafter, some embodiments of the present disclosure will be described in detail with illustrative drawings. When adding reference numerals to components of each drawing, it should be noted that identical components are given the same numerals as much as possible even if they are shown on different drawings. In addition, in the description of the present disclosure, when it is determined that a specific description of a related known configuration or function may obscure the gist of the present disclosure, a detailed description thereof will be omitted.

In the description of the components of an embodiment according to the present disclosure, symbols such as first, second, i), ii), a), b), etc. may be used. These symbols are only intended to distinguish one component from other components, and the nature, order, or sequence of the components is not limited by these symbols. When a part of the specification is said to "include" or "comprise" a component, this means that it may include other components rather than excluding them unless explicitly stated otherwise.

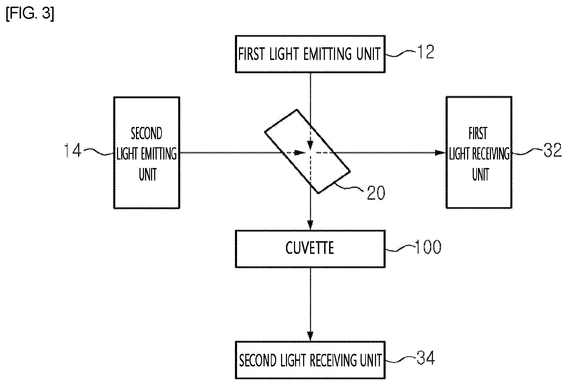

In the present disclosure, the "lower side/downward" refers to a direction from a first light emitting unit 12 toward a cuvette 100 in FIG. 3, and the "upper side/upward" refers to an opposite direction thereof.

In addition, in the present disclosure, the "right side" refers to a direction from a second light emitting unit 14 toward a first light receiving unit 32 in FIG. 3, and the "left side" refers to an opposite direction thereof.

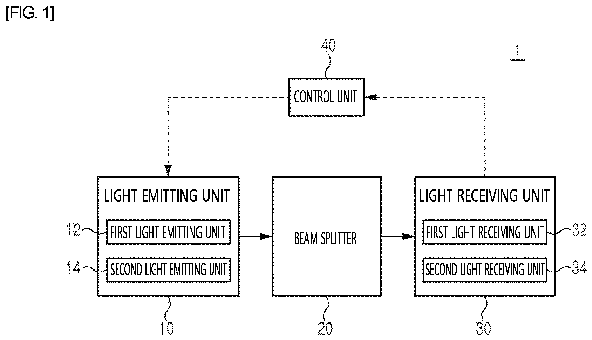

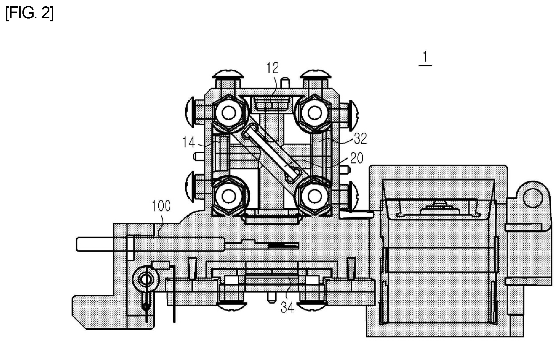

FIG. 1 is a block diagram of a hemoglobin measuring device according to an embodiment of the present disclosure, FIG. 2 illustrates the interior of a hemoglobin measuring device according to an embodiment of the present disclosure, and FIG. 3 illustrates the configuration of a hemoglobin measuring device according to the first embodiment of the present disclosure.

A hemoglobin measuring device 1 according to an embodiment of the present disclosure is configured to measure the concentration of hemoglobin in a sample (that is, blood) using spectrophotometry. For this purpose, the hemoglobin measuring device 1 includes all or some of a light emitting unit 10, a beam splitter 20, a light receiving unit 30, a control unit 40, and a cuvette 100.

The light emitting unit 10 is configured to receive power and emit light toward a sample. In the present disclosure, the light emitting unit 10 may include a first light emitting unit 12 and a second light emitting unit 14.

The first light emitting unit 12 includes a light emitting element and is configured to receive an electric power source and emit light of a wavelength range targeted by a user with a relatively constant output. In the present disclosure, preferably, the wavelength (hereinafter, "first wavelength") of light emitted from the first light emitting unit 12 may be 525 nm to 530 nm.

The first light emitting unit 12 may emit light downwards. However, the present disclosure is not necessarily limited thereto, and light may be emitted upwards depending on the position where the cuvette 100 is placed.

The second light emitting unit 14 includes a light emitting element and is configured to receive an electric power source and emit light of a wavelength range targeted by the user with a relatively constant output. In the present disclosure, preferably, the wavelength (hereinafter, "second wavelength") of light emitted from the second light emitting unit 14 may be 840 nm.

The second light emitting unit 14 may emit light rightwards. However, the present disclosure is not necessarily limited thereto, and light may be emitted leftwards depending on the position where the first light receiving unit 32 is placed.

Meanwhile, the light emitting element used in the light emitting unit 10 may be an LED or photodiode generating a specific wavelength. Alternatively, a white LED and an optical filter assembly transmitting only a specific wavelength may be used.

The beam splitter 20 is configured to propagate at least a part of the incident light in a first direction and the other part thereof in a second direction. In this case, preferably, the second direction may refer to a direction rotated 90 degrees with respect to the first direction. Preferably, the beam splitter 20 is configured to transmit 50% of incident light and refract the remaining 50% in a direction of 90 degrees.

According to the present disclosure, when light of the first wavelength passes through the beam splitter 20, the light may be split into two light beams, one in the downward direction from which it originally proceeds, and the other in the rightward direction refracted at 90 degrees to the original direction from which it originally proceeds.

Similarly, when light of the second wavelength passes through the beam splitter 20, the light may be split into two light beams, one in the rightward direction from which it originally proceeds, and the other in the downward direction refracted at 90 degrees to the original direction from which it originally proceeds.

For this purpose, the beam splitter 20 must be placed at an angle of 45 degrees with respect to the direction in which the light of the first wavelength is emitted.

The light receiving unit 30 is configured to receive light emitted from the light emitting unit 10. For this purpose, the light receiving unit 30 may include a first light receiving unit 32 and a second light receiving unit 34.

The first light receiving unit 32 is placed parallel to the second light emitting unit 14 at the right side of the second light emitting unit 14, and receives light that has passed through the beam splitter 20. That is, the first light receiving unit 32 may receive direct light from the light emitted from the second light emitting unit 14 and refracted light from the light emitted from the first light emitting unit 12.

The first light receiving unit 32 may convert the wavelength of the received light into an electrical signal and transmit the electrical signal to the control unit 40.

The second light receiving unit 34 is placed parallel to the first light emitting unit 12 at the lower side of the first light emitting unit 12, and receives light that has sequentially passed through the beam splitter 20 and cuvette 100. That is, the second light receiving unit 34 may receive direct light from the light emitted from the first light emitting unit 12 and refracted light from the light emitted from the second light emitting unit 14.

The second light receiving unit 34 may convert the wavelength of the received light into an electrical signal and transmit the electrical signal to the control unit 40.

According to the present disclosure, the light received by the first light receiving unit 32 is light before passing through the cuvette 100, and is used to correct the intensity of the light received by the second light receiving unit 34. In addition, the light received by the second light receiving unit 34 is light that passes through the cuvette 100, and is used to analyze a sample in the cuvette 100.

According to the present disclosure, the first light emitting unit 12 and the second light emitting unit 14 are each arranged at 90 degrees, and the first light receiving unit 32 and the second light receiving unit 34 are each arranged at 90 degrees, so that the hemoglobin measuring device 1 may be designed in a space-intensive manner.

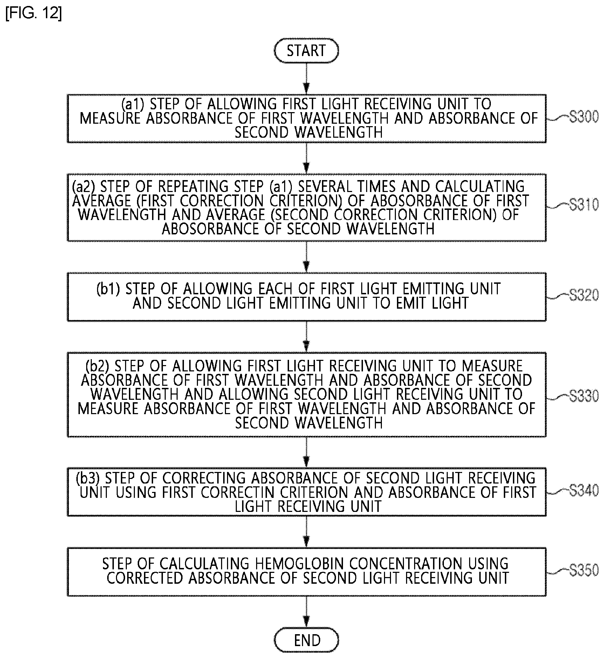

The control unit 40 may transmit a signal to the light emitting unit 10, the signal controlling the amount of power applied to the light emitting unit 10. In addition, the control unit 40 may receive an electrical signal transmitted from the light receiving unit 30 and control a signal applied to the light emitting unit 10 based on the electrical signal. In this regard, a specific method will be described in FIG. 12.

### Description of cuvette 100 according to first embodiment

The cuvette 100 is placed between the beam splitter 20 and the second light receiving unit 34, and is configured to store a sample (that is, blood) therein.

Since light must be transmitted through the cuvette 100, at least a part of the cuvette 100 is made of a transparent material.

The cuvette 100 may be made of glass, but is preferably made of a rigid and optically transparent injection-moldable material such as polystyrene (PS), polyester (PES), polyethylene terephthalate (PET), or polymethyl methacrylate (PMMA). Thus, the cuvette 100 has advantages of enabling mass production, low production costs, and high productivity.

In addition, there is no need to worry about the cuvette 100 breaking, so the cuvette 100 has an advantage of being safe for users to use.

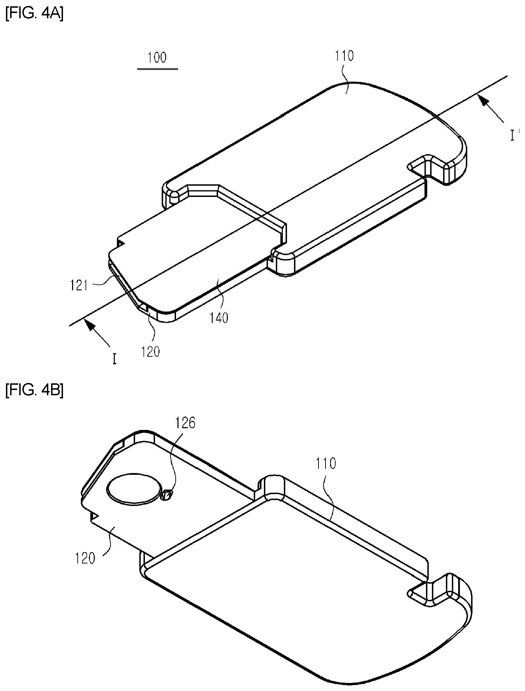

FIG. 4A is a front perspective view of a cuvette used for hemoglobin measurement according to the first embodiment of the present disclosure. FIG. 4B is a bottom perspective view of a cuvette used for hemoglobin measurement according to the first embodiment of the present disclosure.

Referring to FIGS. 4A and 4B, a cuvette 100 according to the first embodiment of the present disclosure includes a handle 110 and a suction unit 120.

The handle 110 is configured for a user to grasp.

The suction unit 120 is formed integrally with the handle 110, and is configured to come into contact with a sample and suck the sample.

When the opening 121 of the suction unit 120 is brought into contact with the sample, the sample may be sucked into the interior of the suction unit 120 by a capillary phenomenon. Since the suction unit 120 is formed to have an internal height of less than 1 mm, it is advantageous in sucking the sample by the capillary phenomenon.

On the bottom surface of the suction unit 120, an air discharge portion 126 formed by penetrating the suction unit 120 may be placed. Air bubbles mixed during sample suction or air bubbles mixed during blood collection may be discharged through the air discharge portion 126. Bubbles contained in a sample may change the light transmittance of the sample. This is considered a major cause of errors in analysis results. Meanwhile, the cuvette 100 according to the present disclosure has the advantage of improving analysis accuracy by discharging such bubbles to the outside of the cuvette 100.

The suction unit 120 has an open upper surface, and a hydrophilic film 140 is attached to the open upper surface, so that an accommodation space may be formed between the suction unit 120 and the hydrophilic film 140.

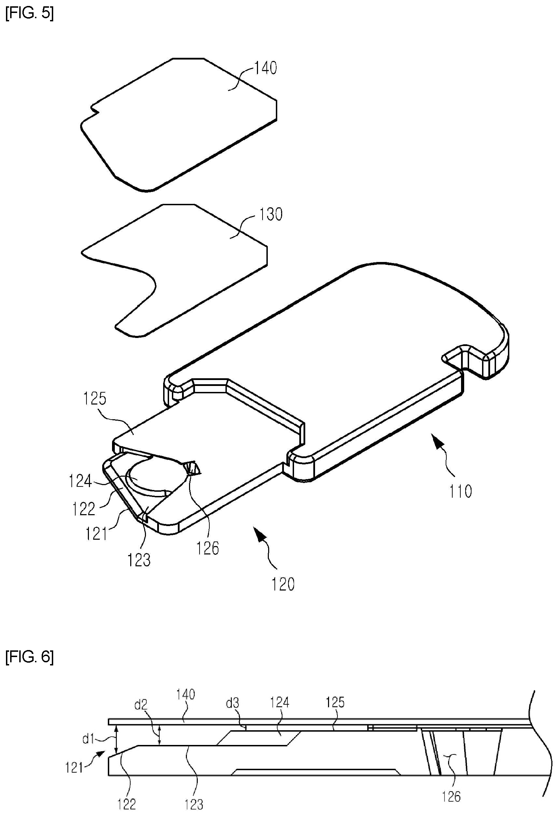

FIG. 5 is an exploded perspective view of a cuvette according to the first embodiment of the present disclosure viewed from the front view. FIG. 6 is an enlarged cross-sectional view of a suction unit taken along the line I-I' in FIG. 4A of the present disclosure.

The configuration of the suction unit 120 and another configuration of the cuvette 100 are described in detail with reference to FIGS. 5 and 6.

The suction unit 120 may further include an inclined surface 122, an inner bottom surface 123, a protrusion 124, and an attachment surface 125.

The inclined surface 122 is formed to extend from the opening 121 toward the inside of the cuvette 100. In this case, it is preferable that the inclined surface 122 be formed so that its height increases in a direction toward the inside of the cuvette 100.

The distance between the starting point of the inclined surface 122 and the hydrophilic film 140, that is, the height (d1) of the opening 121, is preferably 0.3 mm to 1.0 mm.

The distance (d2) between the endpoint of the inclined surface 122 and the hydrophilic film 140 is preferably 0.2 mm to 0.5 mm. That is, as a flow path through which the sample flows is narrowed by the inclined surface 122, the sample may stably diffuse into the inside of the cuvette 100 along the inclined surface 122.

The inner bottom surface 123 is formed to extend from the endpoint of the inclined surface 122 toward the inside of the cuvette 100.

The protrusion 124 is formed to protrude upward from the inner bottom surface 123, and is preferably formed in a circular shape. The distance (d3) between one surface of the protrusion 124 and the hydrophilic film 140 is preferably 0.2 mm or less. Alternatively, the distance (d3) may be 0.05 mm to 0.2 mm. Thus, the sample may be spread thinly enough to allow light to pass through.

In this case, light passing through the beam splitter 20 and proceeding downward passes through the protrusion 124 of the cuvette 100.

The cuvette 100 may further include an adhesive layer 130 and a hydrophilic film 140 in addition to the handle 110 and the suction unit 120.

As described above, the cuvette 100 is made of a hydrophobic material such as polystyrene (PS), polyester (PES), polyethylene terephthalate (PET), or polymethyl methacrylate (PMMA). Thus, it may be difficult to collect blood containing water. In this case, one side of the cuvette 100 may be composed of the hydrophilic film (140), which is a hydrophilic material, thereby improving a capillary phenomenon and the resulting sample collection ability.

Since only one side of the cuvette 100 is made of a hydrophilic material, there are advantages of the overall manufacturing cost of the cuvette 100 being reduced while the sample collection capacity being excellent.

The adhesive layer 130 is configured to attach the suction unit 120 and the hydrophilic film 140 together therebetween.

The adhesive layer 130 is attached to the attachment surface 125 of the suction unit 120, and the hydrophilic film 140 is attached to the adhesive layer 130, so that an accommodation space may be formed between the suction unit 120 and the hydrophilic film 140.

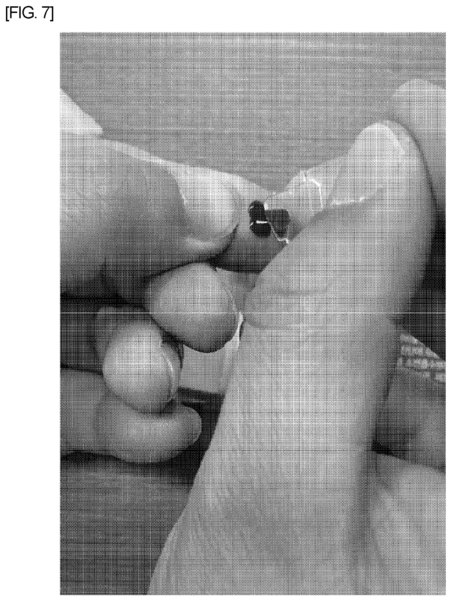

FIG. 7 is a usage status view of a cuvette according to the first embodiment of the present disclosure.

Referring to FIG. 7, it can be seen that at least a part of the user's blood is sucked into the suction unit 120 of the cuvette 100. In this case, the cuvette 100 may collect a relatively small amount of blood due to its small internal volume. Thus, there is an advantage of being user-friendly because the user does not need to collect an excessive amount of its own blood.

### Description of cuvette 200 according to second embodiment

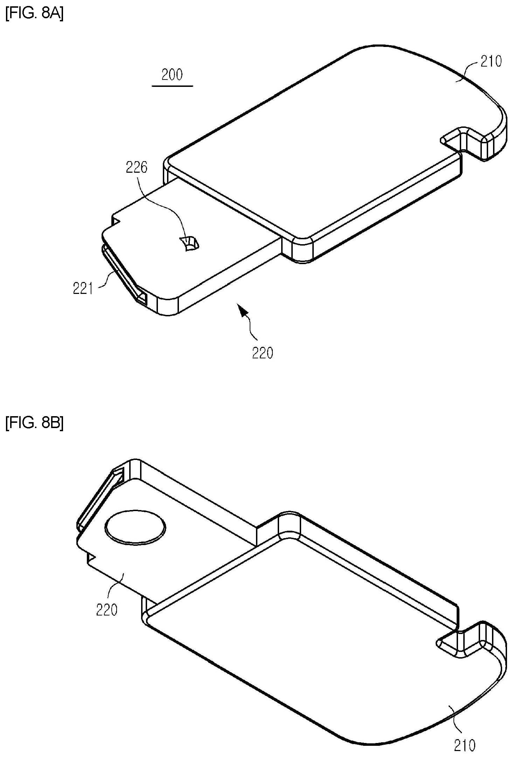

FIG. 8A is a front perspective view of a cuvette according to the second embodiment of the present disclosure. FIG. 7B is a bottom perspective view of a cuvette according to the second embodiment of the present disclosure.

Referring to FIGS. 8A and 8B, a cuvette 200 according to the second embodiment of the present disclosure includes a handle 210 and a suction unit 220.

The handle 210 is configured for a user to grasp.

The suction unit 220 is formed integrally with the handle 210, and is configured to come into contact with a sample and suck the sample.

When the opening 221 of the suction unit 220 is brought into contact with the sample, the sample may be sucked into the interior of the suction unit 220 by a capillary phenomenon. Since the suction unit 220 is formed to have an internal height of less than 1 mm, it is advantageous in sucking the sample by the capillary phenomenon.

On the upper surface of the suction unit 220, an air discharge portion 226 formed by penetrating one surface of the suction unit 220 is placed. The shape and configuration of the air discharge portion 226 according to the second embodiment are the same as those of the air discharge portion 126 according to the first embodiment, so that the related description is replaced by the description of the air discharge portion 126.

The suction unit 220 according to the second embodiment of the present disclosure is in a form in which its upper surface, lower surface, and side surfaces are all closed and it is open toward the opening 221, and an accommodation space may be formed inside the suction unit 220 itself.

At least a part of the suction unit 220 may be made of a hydrophilic bio-fiber material. Here, the hydrophilic bio-fiber material is, for example, a material formed by acetylating a hydroxyl group (-OH) of cellulose, which is a polysaccharide fiber. Since the hydrophilic biofiber material is relatively inexpensive, the manufacturing cost can be reduced, while capture capacity remains excellent.

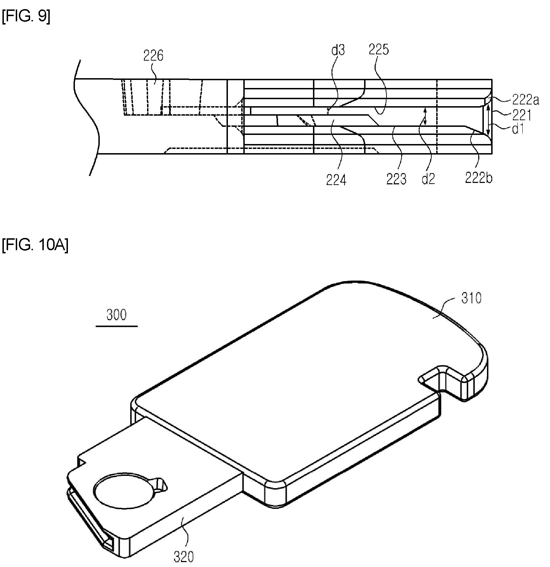

FIG. 9 is a left side view of a cuvette according to the second embodiment of the present disclosure.

The configuration of the suction unit 220 is described in detail with reference to FIG. 9.

The suction unit 220 may further include a first inclined surface 222a, a second inclined surface 222b, an inner bottom surface 223, a protrusion 224, and an inner upper surface 225.

The first inclined surface 222a and the second inclined surface 222b are formed to extend from the opening 221 toward the inside of the cuvette 200. In this case, it is preferable that the first inclined surface 222a and the second inclined surface 222b be formed so that their heights increase in a direction toward the inside of the cuvette 200.

The distance (d1) between the first inclined surface 222a and the second inclined surface 222b is preferably 0.3 mm to 1.0 mm.

The inner bottom surface 223 is formed to extend from the endpoint of the first inclined surface 222a toward the inside of the cuvette 200.

The protrusion 224 is formed to protrude upward from the inner bottom surface 223, and is preferably formed in a circular shape. In this case, light passing through the beam splitter 20 and proceeding downward passes through the protrusion 224 of the cuvette 200.

The inner upper surface 225 is formed to extend from the endpoint of the second inclined surface 222b toward the inside of the cuvette 200.

The distance (d2) between the inner bottom surface 223 and the inner upper surface 225 is preferably 0.2 mm to 0.5 mm. In addition, the distance (d3) between the protrusion 224 and the inner upper surface 225 is preferably 0.2 mm or less. Alternatively, the distance (d3) may be 0.05 mm to 0.2 mm. Thus, the sample may be spread thinly enough to allow light to pass through.

That is, since the size decreases as it goes to d1, d2, and d3, the path through which the sample flows becomes narrower, thereby allowing the sample to stably diffuse into the cuvette 200.

### Description of cuvette 300 according to third embodiment

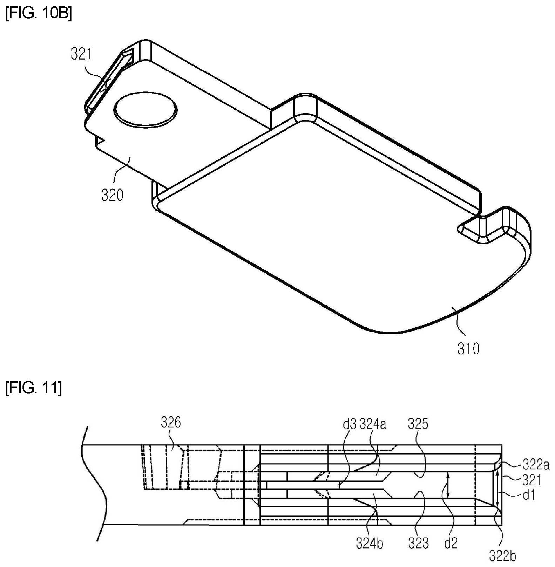

FIG. 10A is a front perspective view of a cuvette according to the third embodiment of the present disclosure. FIG. 10B is a bottom perspective view of a cuvette according to the third embodiment of the present disclosure.

Referring to FIGS. 10A and 10B, a cuvette 300 according to the third embodiment of the present disclosure includes a handle 310 and a suction unit 320.

The handle 310 is configured for a user to grasp.

The suction unit 320 is formed integrally with the handle 310, and is configured to come into contact with a sample and suck the sample.

When the opening 321 of the suction unit 320 is brought into contact with the sample, the sample may be sucked into the interior of the suction unit 320 by a capillary phenomenon. Since the suction unit 320 is formed to have an internal height of less than 1 mm, it is advantageous in sucking the sample by the capillary phenomenon.

On the upper surface of the suction unit 320, an air discharge portion 326 formed by penetrating one surface of the suction unit 320 is placed. The shape and configuration of the air discharge portion 326 according to the third embodiment are the same as those of the air discharge portion 126 according to the first embodiment, so that the related description is replaced by the description of the air discharge portion 126.

The suction unit 320 according to the third embodiment of the present disclosure is in a form in which its upper surface, lower surface, and side surfaces are all closed and it is open toward the opening 321, and an accommodation space may be formed inside the suction unit 320 itself.

At least a part of the suction unit 320 may be made of a hydrophilic bio-fiber material. A description related to the material of the suction unit 320 according to the third embodiment is replaced with the description of the suction unit 220 according to the second embodiment.

FIG. 11 is a left side view of a cuvette according to the third embodiment of the present disclosure.

The configuration of the suction unit 320 is described in detail with reference to FIG. 11.

The suction unit 320 may further include a first inclined surface 332a, a second inclined surface 332b, an inner bottom surface 323, a first protrusion 324a, a second protrusion 324b, and an inner upper surface 325.

The shape and effect of the first inclined surface 332a and the second inclined surface 332b according to the third embodiment are the same as those of the first inclined surface 222a and the second inclined surface 222b according to the second embodiment, so the description of the first inclined surface 332a and the second inclined surface 332b is replaced by the description of the first inclined surface 222a and the second inclined surface 222b.

The inner bottom surface 323 is formed to extend from the endpoint of the first inclined surface 332a toward the inside of the cuvette 300.

The first protrusion 324a is formed to protrude upward from the inner bottom surface 323, and is preferably formed in a circular shape.

The inner upper surface 325 is formed to extend from the endpoint of the second inclined surface 332b toward the inside of the cuvette 300.

The second protrusion 324b is formed to protrude downward from the inner upper surface 325, and is preferably formed in a circular shape. In this case, it is preferable that the second protrusion 324b and the first protrusion 324a be formed in the same shape and be arranged to face each other.

Light passing through the beam splitter 20 and proceeding downward passes through both the first protrusion 324a and second protrusion 324b of the cuvette 300.

The distance (d2) between the inner bottom surface 323 and the inner upper surface 325 is preferably 0.2 mm to 0.5 mm. In addition, the distance (d3) between the first protrusion 324a and the second protrusion 324b is preferably 0.2 mm or less. Alternatively, the distance (d3) may be 0.05 mm to 0.2 mm. Thus, the sample may be spread thinly enough to allow light to pass through.

That is, since the size decreases as it goes to d1, d2, and d3, the path through which the sample flows becomes narrower, thereby allowing the sample to stably diffuse into the cuvette 300.

### Method of measuring hemoglobin using hemoglobin measuring device 1

FIG. 12 is a flow chart of a method of measuring hemoglobin using a hemoglobin measuring device according to an embodiment of the present disclosure.

A method of measuring hemoglobin using a hemoglobin measuring device 1 according to an embodiment of the present disclosure is described with reference to FIG. 12.

The first light receiving unit 32 measures the absorbance (A1_REF) of a first wavelength and the absorbance (A2_REF) of a second wavelength (S300).

The step S300 is repeated several times, and the average of the absorbance (A1_REF) of the first wavelength and the average of the absorbance (A2_REF) of the second wavelength are calculated (S310). In this case, the average of the absorbance (A1_REF) of the first wavelength is referred to as a first correction criterion, and the average of the absorbance (A2_REF) of the second wavelength is referred to as a second correction criterion.

Table 1 summarizes the absorbances measured according to the steps S300 to S310 and the hemoglobin concentrations calculated using the same. Referring to Table 1, the first correction criterion is 2469.9, and the second correction criterion is 4000.3.

**[Table 1]**

| Order | A1 | A1_REF | A2 | A2_REF | Hemoglobin concentration (g/dL) |
|---|---|---|---|---|---|
| 1 | 1221 | 2471 | 1301 | 4001 | 14.7 |
| 2 | 1222 | 2471 | 1301 | 4001 | 14.7 |
| 3 | 1222 | 2471 | 1301 | 4000 | 14.7 |
| 4 | 1221 | 2469 | 1300 | 3999 | 14.7 |
| 5 | 1220 | 2470 | 1301 | 4000 | 14.7 |
| 6 | 1221 | 2469 | 1301 | 4001 | 14.7 |
| 7 | 1222 | 2470 | 1302 | 4000 | 14.7 |
| 8 | 1221 | 2469 | 1301 | 4000 | 14.7 |
| 9 | 1221 | 2470 | 1301 | 4000 | 14.7 |
| 10 | 1221 | 2469 | 1302 | 4001 | 14.7 |
| Average | 1221.2 | **2469.9** | 1301.1 | **4000.3** | 14.7 |

| | | | | | |
|---|---|---|---|---|---|
| A1: Absorbance of the first wavelength measured by the second light receiving unit A1_REF: Absorbance of the first wavelength measured by the first light receiving unit A2: Absorbance of the second wavelength measured by the second light receiving unit A2_REF: Absorbance of the second wavelength measured by the first light receiving unit | | | | | |

Light is emitted from each of the first light emitting unit 12 and the second light emitting unit 14 (S320).

The first light receiving unit 32 measures the absorbance (A1_REF) of the first wavelength and the absorbance (A2_REF) of the second wavelength, and the second light receiving unit 34 measures the absorbance (A1) of the first wavelength and the absorbance (A2) of the second wavelength (S330). Each measured absorbance is converted into an electrical signal and transmitted to the control unit 40.

The control unit 40 calculates the corrected absorbances (A1_corrected and A2_corrected) of the second light receiving unit 34 using the first correction criterion and the absorbances (A1_REF and A2_REF) of the first light receiving unit (S340).

In this case, the corrected absorbances (A1_corrected and A2_corrected) of the second light receiving unit 34 are calculated using Equation 1 and Equation 2. [Equation 1] $A 1 _ corrected = A 1 \times \frac{first correction criterion}{A 1 REF}$ [Equation 2] $A 2 _ corrected = A 2 \times \frac{second correction criterion}{A 2 REF}$

The concentration of hemoglobin in a sample in the cuvette 100 is calculated using the corrected absorbances (A1_corrected and A2_corrected) of the second light receiving unit 34 (S350). In this case, the concentration of hemoglobin is calculated using Equation 3. [Equation 3] $Hemoglobin concentration = a \times ln \left(A1_corrected/A2_corrected\right) + b$

In this case, a is a constant that determines discrimination power according to hemoglobin concentration, and may be determined through an empirical formula. In addition, b is a correction constant, and may be determined through an empirical formula. Meanwhile, in the present disclosure, the hemoglobin concentration was calculated assuming that a is 10.659 and b is 15.388.

When initially used, a light emitting element may emit light of a target wavelength corresponding to applied power at a relatively constant intensity. However, when repeatedly used, the light emitting element deteriorates, and produces lower output for the same power compared to when initially used. According to the present disclosure, there is an advantage in that the output reduction due to deterioration of the light emitting element can be corrected by correcting the amount (that is, absorbance) of light received by the second light receiving unit 34 using the light received by the first light receiving unit 32. The accuracy of the method of measuring hemoglobin according to an embodiment of the present disclosure is further described with reference to Table 2 and Table 3.

Table 2 shows the results of measurements of hemoglobin concentration using a hemoglobin measuring device that has performed a preset number of measurement cycles, and summarizes a case where no correction using the first light receiving unit 32 was performed. Meanwhile, the experimental results according to Table 2 were performed under the same conditions (that is, same power supply) as the experimental results according to Table 1.

**[Table 2]**

| Order | A1 | A1_REF | A2 | A2_REF | Hemoglobin concentration (g/dL) |
|---|---|---|---|---|---|
| 1 | 1217 | 2469 | 1240 | 3860 | 15.2 |
| 2 | 1216 | 2469 | 1239 | 3861 | 15.2 |
| 3 | 1216 | 2469 | 1239 | 3860 | 15.2 |
| 4 | 1218 | 2470 | 1241 | 3860 | 15.2 |
| 5 | 1216 | 2468 | 1240 | 3861 | 15.2 |
| 6 | 1216 | 2468 | 1237 | 3862 | 15.2 |
| 7 | 1217 | 2469 | 1241 | 3862 | 15.2 |
| 8 | 1217 | 2469 | 1240 | 3861 | 15.2 |
| 9 | 1218 | 2468 | 1241 | 3860 | 15.2 |
| 10 | 1217 | 2469 | 1240 | 3862 | 15.2 |
| Average | 1216.8 | 2468.8 | 1239.8 | 3860.9 | **15.2** |

| | | | | | |
|---|---|---|---|---|---|
| A1: Absorbance of the first wavelength measured by the second light receiving unit A1_REF: Absorbance of the first wavelength measured by the first light receiving unit A2: Absorbance of the second wavelength measured by the second light receiving unit A2_REF: Absorbance of the second wavelength measured by the first light receiving unit | | | | | |

Referring to Table 2, it was found that the hemoglobin concentration was 15.2 g/dL, which was significantly different from the actual hemoglobin concentration of 14.7 g/dL.

Table 3 shows the results of measurements of hemoglobin concentration using a hemoglobin measuring device that has performed a preset number of measurement cycles, and summarizes a case where correction using the first light receiving unit 32 was performed. Meanwhile, the experimental results according to Table 3 were performed under the same conditions (that is, the same power supply) as the experimental results according to Table 1.

**[Table 3]**

| Order | A1 | A1_correct ed | A1_ref | A2 | A2_correct ed | A2_ref | Hemoglobin concentratio n (g/dL) |
|---|---|---|---|---|---|---|---|
| 1 | 1217 | 1217.4 | 2469 | 1240 | 1285.1 | 3860 | 14.8 |
| 2 | 1216 | 1216.4 | 2469 | 1239 | 1283.7 | 3861 | 14.8 |
| 3 | 1216 | 1216.4 | 2469 | 1239 | 1284.0 | 3860 | 14.8 |
| 4 | 1218 | 1218.0 | 2470 | 1241 | 1286.1 | 3860 | 14.8 |
| 5 | 1216 | 1216.9 | 2468 | 1240 | 1284.7 | 3861 | 14.8 |
| 6 | 1216 | 1216.9 | 2468 | 1237 | 1281.3 | 3862 | 14.8 |
| 7 | 1217 | 1217.4 | 2469 | 1241 | 1285.4 | 3862 | 14.8 |
| 8 | 1217 | 1217.4 | 2469 | 1240 | 1284.7 | 3861 | 14.8 |
| 9 | 1218 | 1218.9 | 2468 | 1241 | 1286.1 | 3860 | 14.8 |
| 10 | 1217 | 1217.4 | 2469 | 1240 | 1284.4 | 3862 | 14.8 |
| Averag e | 1216.8 | 1217.3 | 2468.8 | 1239.8 | 1284.6 | 3860.9 | 14.8 |

| | | | | | | | |
|---|---|---|---|---|---|---|---|
| A1: Absorbance of the first wavelength measured by the second light receiving unit A1_corrected: corrected value of absorbance of the first wavelength measured by the second light receiving unit A1_REF: Absorbance of the first wavelength measured by the first light receiving unit A2: Absorbance of second wavelength measured by second light receiving unit A2_corrected: corrected value of absorbance of the second wavelength measured by the second light receiving unit A2_REF: Absorbance of the second wavelength measured by the first light receiving unit | | | | | | | |

Referring to Table 3, it can be found that the hemoglobin concentration is 14.8 g/dL, which shows an error rate of 0.68% compared to the actual hemoglobin concentration of 14.7 g/dL, indicating a highly reliable result.

FIG. 2 illustrates the interior of a hemoglobin measuring device according to an embodiment of the present disclosure. FIG. 2 is an example of an actual hemoglobin measuring device 1, and it should be noted that a configuration other than the configuration described in the above contents may be appropriately design-changed by the user.

As shown in FIG. 2, the first light emitting unit 12 and the second light emitting unit 14 are each arranged at 90 degrees, and the first light receiving unit 32 and the second light receiving unit 34 are also each arranged at 90 degrees, so that the hemoglobin measuring device 1 may be designed in a space-intensive manner.

FIG. 4A is a front perspective view of a cuvette used for hemoglobin measurement according to the first embodiment of the present disclosure. FIG. 4B is a bottom perspective view of a cuvette used for hemoglobin measurement according to the first embodiment of the present disclosure.

Referring to FIGS. 4A and 4B, a cuvette 100 according to the first embodiment of the present disclosure includes a handle 110 and a suction unit 120.

The handle 110 is configured for a user to grasp.

The suction unit 120 is configured to suck a sample when it comes into contact with the sample. Specifically, when the opening 121 of the suction unit 120 is brought close to the sample, the sample is sucked into the interior of the suction unit 120 by a capillary phenomenon. Since the cuvette 100 is formed with a thickness of less than 1 mm, it is advantageous for sample suction. Specifically, the opening 121 is formed with a thickness of about 0.2 mm to 0.5 mm, and the surface contact angle is 20 degrees or less, so it has an advantage of having a relatively large suction power even with a simple structure.

It will be appreciated by those skilled in the art that the present invention as described above may be implemented into other specific forms without departing from the technical spirit thereof or essential characteristics. Thus, it is to be appreciated that embodiments described above are intended to be illustrative in all aspects, and not restrictive. The scope of the present invention is represented by the appended claims to be described below rather than the detailed description, and it is to be interpreted that the meaning and scope of the appended claims and all the changes or modified forms derived from equivalents thereof come within the scope of the present invention.

### (Description of Reference Numerals)

1: Hemoglobin measuring device
10: Light emitting unit
12: First light emitting unit
14: Second light emitting unit
20: Beam splitter
30: Light receiving unit
32: First light receiving unit
34: Second light receiving unit
40: Control unit
100, 200, 300: Cuvette
110, 210, 310: Handle
120, 220, 320: Suction unit
130: Hydrophilic film
140: Adhesive layer

## Claims

1. A cuvette 100 configured to collect a sample, the cuvette 100 comprising:
a handle 110 configured to grasp;
a suction unit 120 formed integrally with the handle 110, having an open upper surface and configured to suck a sample through an opening 121 formed at one end;
a hydrophilic film 140 attached to the suction unit 120 to form an accommodation space together with the suction unit 120; and
an adhesive layer 130 placed between the hydrophilic film 140 and an attachment surface 125 of the suction unit 120 to attach both of them.

2. The cuvette of claim 1,
wherein the suction unit 120 is made of one or more materials selected from the group consisting of polystyrene (PS), polyester (PES), polyethylene terephthalate (PET), and polymethyl methacrylate (PMMA).

3. The cuvette of claim 1,
wherein the suction unit 120 comprises:
an inclined surface 122 formed to extend from the opening 121 toward the inside of the cuvette 100;
an inner bottom surface 123 formed to extend from an endpoint of the inclined surface 122 toward the inside of the cuvette 100;
a circular protrusion 124 formed to protrude upward from the inner bottom surface 123; and
an air discharge portion 126 formed to penetrate the suction unit 120.

4. The cuvette of claim 3,
wherein the height of the opening 121, that is, the distance (d1) between the hydrophilic film 140 and a starting point of the inclined surface 122 ranges from 0.3 mm to 1.0 mm,
the distance (d2) between the hydrophilic film 140 and the endpoint of the inclined surface 122 ranges from 0.2 mm to 0.5 mm, and
the distance (d3) between the protrusion 124 and the hydrophilic film 140 ranges from 0.05 mm to 0.2 mm.

5. A cuvette 200 or 300 configured to capture a sample, the cuvette 200 or 300 comprising:
a handle 210 or 310 configured to grasp; and
a suction unit 220 or 320 formed integrally with the handle 210 or 310 and configured to suck a sample through an opening 221 or 321 formed at one end,
wherein the suction unit 220 or 320 has a shape in which the upper surface, lower surface, and both side surfaces are closed, and at least a part of the suction unit 220 or 320 is made of a hydrophilic bio-fiber material.

6. The cuvette of claim 5,
wherein the suction unit 220 comprises:
a first inclined surface 222a formed at one end of an inner bottom surface 223 of the suction unit 220 and extending from the opening 221 toward the inside of the cuvette 200;
a second inclined surface 222b formed at one end of an inner upper surface 225 of the suction unit 220 and extending from the opening 221 toward the inside of the cuvette 200;
a circular protrusion 224 formed to protrude upward from the inner bottom surface 223; and
an air discharge portion 226 formed to penetrate the upper surface of the suction unit 220.

7. The cuvette of claim 5,
wherein the suction unit 320 comprises:
a first inclined surface 322a formed at one end of an inner bottom surface 323 of the suction unit 320 and extending from the opening 321 toward an inside of the cuvette 300;
a second inclined surface 322b formed at one end of an inner upper surface 325 of the suction unit 320 and extending from the opening 321 toward the inside of the cuvette 300;
a first circular protrusion 324a formed to protrude upward from the inner bottom surface 323;
a second circular protrusion 324b formed to protrude downward from the inner upper surface 325; and
an air discharge portion 326 formed to penetrate the upper surface of the suction unit 320.

8. A hemoglobin measuring device using the cuvette 100, 200, or 300 of any one of claims 1 to 7, the hemoglobin measuring device comprising:
a first light emitting unit 12 configured to emit light downward;
a second light emitting unit 14 configured to emit light rightwards;
a first light receiving unit 32 arranged parallel to the second light emitting unit 14 at the right side of the second light emitting unit 14;
a second light receiving unit 34 arranged parallel to the first light emitting unit 12 at the lower side of the first light emitting unit 12; and
a beam splitter 20 placed at the point where a path from the first light emitting unit 12 to the second light receiving unit 34 intersects with the path from the second light emitting unit 14 to the first light receiving unit 32,
wherein the cuvette 100, 200, or 300 is placed between the beam splitter 20 and the second light receiving unit 34.

9. The hemoglobin measuring device of claim 8,
wherein the wavelength of light emitted from the first light emitting unit 12 ranges from 525 nm to 530 nm, and
a wavelength of light emitted from the second light emitting unit 14 is 840 nm.

10. A method of measuring hemoglobin concentration using a hemoglobin measuring device 1 comprising a first light emitting unit 12, a second light emitting unit 14, a first light receiving unit 32 receiving a part of light emitted from the first light emitting unit 12 and a part of light emitted from the second light emitting unit 14, and a second light receiving unit 34 receiving another part of light emitted from the first light emitting unit 12 and a part of light emitted from the second light emitting unit 14, the method comprising:
(A) a step of calculating a first correction criterion and a second correction criterion using the hemoglobin measuring device 1 used a number of times less than a preset number of times; and
(B) a step of measuring the concentration of hemoglobin in blood using the hemoglobin measuring device 1 used more than a preset number of times.

11. The method of claim 10,
wherein the step (A) comprises:
(a1) a step of allowing the first light receiving unit 32 to measure the absorbance (A1_REF) of a wavelength (first wavelength) of light emitted from the first light emitting unit 12 and the absorbance (A2_REF) of a wavelength (second wavelength) of light emitted from the second light emitting unit 14;
(a21) a step of repeating the step (a1) several times; and
(a22) a step of allowing a control unit 40 to calculate a first correction criterion which is an average of the absorbance (A1_REF) of the first wavelength and a second correction criterion which is an average of the absorbance (A2_REF) of the second wavelength.

12. The method of claim 11,
wherein the step (B) comprises:
(b1) a step of allowing each of the first light emitting unit 12 and the second light emitting unit 14 to emit light;
(b2) a step of allowing the first light receiving unit 32 to measure the absorbance (A1_REF) of the first wavelength and the absorbance (A2_REF) of the second wavelength and allowing the second light receiving unit 34 to measure the absorbance (A1) of the first wavelength and the absorbance (A2) of the second wavelength;
(b3) a step of allowing the control unit 40 to calculate corrected absorbances (A1_corrected and A2_corrected) of the second light receiving unit 34 using the first correction criterion calculated in the step (a22) and the absorbances (A1_REF and A2_REF) of the first light receiving unit 32 measured in the step (b2); and
(b4) a step of calculating the concentration of hemoglobin of a sample in the cuvette 100 using the corrected absorbances (A1_corrected and A2_corrected) of the second light receiving unit 34.

13. The method of claim 12,
wherein, in the step (b3), the corrected absorbances (A1_corrected and A2_corrected) of the second light receiving unit 34 are calculated using Equation 1 and Equation 2 below: [Equation 1] $A 1 _ corrected = A 1 \times \frac{first correction criterion}{A 1 REF}$ [Equation 2] $A 2 _ corrected = A 2 \times \frac{second correction criterion}{A 2 REF}$

14. The method of claim 13,
wherein, in the step (b4), the concentration of hemoglobin in the sample in the cuvette 100 is calculated using the corrected absorbances (A1_corrected and A2_corrected) of the second light receiving unit 34 and Equation 3 below: [Equation 3] $Hemoglobin concentration = a \times ln \left(A1_corrected/A2_corrected\right) + b$ here, a is a constant that determines discrimination power according to hemoglobin concentration, and b is a correction constant.

15. The method of claim 11,
wherein the first wavelength ranges from 525 nm to 530 nm, and
the second wavelength is 840 nm.
